# EUROPEAN PATENT APPLICATION

(11) **EP 1 075 841 A1**
(43) Date of publication of application: **14.02.2001**
(21) Application number: 99202640.1
(22) Date of filing: 13.08.1999
(51) Int. Cl.: A61K 39/09, C07K 14/315, C12N 15/31, C07K 16/12, A61P 31/04

(54) **Pneumococcal vaccines**

(71) Applicant: Erasmus Universiteit Rotterdam, 3015 GE Rotterdam (NL)
(72) Inventor: de Groot, Ronald, 3045 CG Rotterdam (NL); Hermans, Peter Wilhelmus Maria, 2807 EJ Gouda (NL)
(74) Representative: Ottevangers, Sietse Ulbe

(57) **Abstract**

The invention relates to the use of a protein or a fragment thereof of *S. pneumoniae,* its use for the preparation of a vaccine for the preventive treatment of a *S. pneumoniae* infection, compositions comprising protease maturation protein of *S. pneumoniae* or a fragment thereof, vaccines comprising said protein or fragment thereof, use of a nucleic acid sequence encoding for said protein or fragment thereof, vectors wherein the nucleic acid sequence is brought to expression and to recombinant protease maturation protein or a fragment thereof.

### Annex to the application documents - subsequently filed sequences listing

SEQUENCE LISTING

<110> Erasmus Universiteit Rotterdam

<120> Pneumococcal vaccines

<130> P50337EP00

<140> 99202640.1
<141> 1999-08-13

<160> 5

<170> PatentIn Ver. 2.1

<210> 1
<211> 966
<212> DNA
<213> Streptococcus pneumoniae

<220>
<223> /note="The S. pneumoniae nucleotides 7632-8597 on
      contig 33"

<400> 1
agtaacactt atctcaaagg agtagacatg aagaaaaaat tattggcagg tgccatcaca 60
ctattatcag tagcaacttt agcagcttgt tcgaaagggt cagaaggtgc agaccttatc 120
agcatgaaag gggatgtcat tacagaacat caattttatg agcaagtgaa aagcaaccct 180
tcagcccaac aagtcttgtt aaatatgacc atccaaaaag tttttgaaaa acaatatggc 240
tcagagcttg atgataaaga ggttgatgat actattgccg aagaaaaaaa acaatatggc 300
gaaaactacc aacgtgtctt gtcacaagca ggtatgactc ttgaaacacg taaagctcaa 360
attcgtacaa gtaaattagt tgagttggca gttaagaagg tagcagaagc tgaattgaca 420
gatgaagcct ataagaaagc ctttgatgag tacactccag atgtaacggc tcaaatcatc 480
cgtcttaata atgaagataa ggccaaagaa gttctcgaaa aagccaaggc agaaggtgct 540
gattttgctc aattagccaa agataattca actgatgaaa aaacaaaaga aaatggtgga 600
gaaattacct ttgattctgc ttcaacagaa gtacctgagc aagtcaaaaa agccgctttc 660
gctttagatg tggatggtgt ttctgatgtg attacagcaa ctggcacaca agcctacagt 720
agccaatatt acattgtaaa actcactaag aaaacagaaa aatcatctaa tattgatgac 780
tacaaagaaa aattaaaaac tgttatcttg actcaaaaac aaaatgattc aacatttgtt 840
caaagcatta tcggaaaaga attgcaagca gccaatatca aggttaagga ccaagccttc 900
caaaatatct ttacccaata tatcggtggt ggagattcaa gctcaagcag tagtacatca 960
aacgaa                                                            966


<210> 2
<211> 322
<212> PRT
<213> Streptococcus pneumoniae

<220>
<223> /note="The encoding polypeptide sequence harboring
      Pmp"

<400> 2
Ser Asn Thr Tyr Leu Lys Gly Val Asp Met Lys Lys Lys Leu Leu Ala
  1               5                  10                  15

Gly Ala Ile Thr Leu Leu Ser Val Ala Thr Leu Ala Ala Cys Ser Lys
             20                  25                  30

Gly Ser Glu Gly Ala Asp Leu Ile Ser Met Lys Gly Asp Val Ile Thr
         35                  40                  45

Glu His Gln Phe Tyr Glu Gln Val Lys Ser Asn Pro Ser Ala Gln Gln
     50                  55                  60

Val Leu Leu Asn Met Thr Ile Gln Lys Val Phe Glu Lys Gln Tyr Gly
65                  70                  75                  80

Ser Glu Leu Asp Asp Lys Glu Val Asp Asp Thr Ile Ala Glu Glu Lys
                85                  90                  95

Lys Gln Tyr Gly Glu Asn Tyr Gln Arg Val Leu Ser Gln Ala Gly Met
            100                 105                 110

Thr Leu Glu Thr Arg Lys Ala Gln Ile Arg Thr Ser Lys Leu Val Glu
        115                 120                 125

Leu Ala Val Lys Lys Val Ala Glu Ala Glu Leu Thr Asp Glu Ala Tyr
    130                 135                 140

Lys Lys Ala Phe Asp Glu Tyr Thr Pro Asp Val Thr Ala Gln Ile Ile
145                 150                 155                 160

Arg Leu Asn Asn Glu Asp Lys Ala Lys Glu Val Leu Glu Lys Ala Lys
                165                 170                 175

Ala Glu Gly Ala Asp Phe Ala Gln Leu Ala Lys Asp Asn Ser Thr Asp
            180                 185                 190

Glu Lys Thr Lys Glu Asn Gly Gly Glu Ile Thr Phe Asp Ser Ala Ser
        195                 200                 205

Thr Glu Val Pro Glu Gln Val Lys Lys Ala Ala Phe Ala Leu Asp Val
    210                 215                 220

Asp Gly Val Ser Asp Val Ile Thr Ala Thr Gly Thr Gln Ala Tyr Ser
225                 230                 235                 240

Ser Gln Tyr Tyr Ile Val Lys Leu Thr Lys Lys Thr Glu Lys Ser Ser
                245                 250                 255

Asn Ile Asp Asp Tyr Lys Glu Lys Leu Lys Thr Val Ile Leu Thr Gln
            260                 265                 270

Lys Gln Asn Asp Ser Thr Phe Val Gln Ser Ile Ile Gly Lys Glu Leu
        275                 280                 285

Gln Ala Ala Asn Ile Lys Val Lys Asp Gln Ala Phe Gln Asn Ile Phe
    290                 295                 300

Thr Gln Tyr Ile Gly Gly Gly Asp Ser Ser Ser Ser Ser Ser Thr Ser
305                 310                 315                 320

Asn Glu

<210> 3
<211> 299
<212> PRT
<213> Lactobacillus paracasei

<220>
<223> /note="The protease maturation protein sequence of
      Lactobacillus paracasei"

<400> 3
Met Lys Lys Lys Met Arg Leu Lys Val Leu Leu Ala Ser Thr Ala Thr
1               5                   10                  15

Ala Leu Leu Leu Leu Ser Gly Cys Gln Ser Asn Gln Ala Asp Gln Lys

20                  25                  30

Val Ala Thr Tyr Ser Gly Gly Lys Val Thr Glu Ser Asn Phe Tyr Lys
        35                  40                  45

Glu Leu Lys Gln Ser Pro Thr Thr Lys Thr Met Leu Ala Asn Met Leu
    50                  55                  60

Ile Tyr Arg Ala Leu Asn His Ala Tyr Gly Lys Ser Val Ser Thr Lys
65                  70                  75                  80

Thr Val Asn Asp Ala Tyr Asp Ser Tyr Lys Gln Gln Tyr Gly Glu Asn
                85                  90                  95

Phe Asp Ala Phe Leu Ser Gln Asn Gly Phe Ser Arg Ser Ser Phe Lys
            100                 105                 110

Glu Ser Leu Arg Thr Asn Phe Leu Ser Glu Val Ala Leu Lys Lys Leu
        115                 120                 125

Lys Lys Val Ser Glu Ser Gln Leu Lys Ala Val Trp Lys Thr Tyr Gln
    130                 135                 140

Pro Lys Val Thr Val Gln His Ile Leu Thr Ser Asp Glu Asp Thr Ala
145                 150                 155                 160

Lys Gln Val Ile Ser Asp Leu Ala Ala Gly Lys Asp Phe Ala Thr Leu
                165                 170                 175

Ala Lys Thr Asp Ser Ile Asp Thr Ala Thr Lys Asp Asn Gly Gly Lys
            180                 185                 190

Ile Ser Phe Glu Ser Asn Asn Lys Thr Leu Asp Ala Thr Phe Lys Asp
        195                 200                 205

Ala Ala Tyr Lys Leu Lys Asn Gly Asp Tyr Thr Gln Thr Pro Val Lys
    210                 215                 220

Val Thr Asn Gly Tyr Glu Val Ile Lys Met Ile Asn His Pro Ala Lys
225                 230                 235                 240

Gly Thr Phe Thr Ser Ser Lys Lys Ala Leu Thr Ala Ser Val Tyr Ala
                245                 250                 255

Lys Trp Ser Arg Asp Ser Ser Ile Met Gln Arg Val Ile Ser Gln Val
            260                 265                 270

Leu Lys Asn Gln His Val Thr Ile Lys Asp Lys Asp Leu Ala Asp Ala
        275                 280                 285

Leu Asp Ser Tyr Lys Lys Pro Ala Thr Thr Asn
    290                 295


<210> 4
<211> 299
<212> PRT
<213> Lactococcus lactis

<220>
<223> /note="The protease maturation protein sequence of
      Lactococcus lactis subspec. lactis"

<400> 4
Met Lys Lys Lys Met Arg Leu Lys Val Leu Leu Ala Ser Thr Ala Thr
1               5                   10                  15

Ala Leu Leu Leu Leu Ser Gly Cys Gln Ser Asn Gln Thr Asp Gln Thr
            20                  25                  30

Val Ala Thr Tyr Ser Gly Gly Lys Val Thr Glu Ser Ser Phe Tyr Lys
        35                  40                  45

Glu Leu Lys Gln Ser Pro Thr Thr Lys Thr Met Leu Ala Asn Met Leu
    50                  55                  60

Ile Tyr Arg Ala Leu Asn His Ala Tyr Gly Lys Ser Val Ser Thr Lys
65                  70                  75                  80

Thr Val Asn Asp Ala Tyr Asp Ser Tyr Lys Gln Gln Tyr Gly Glu Asn
                85                  90                  95

Phe Asp Ala Phe Leu Ser Gln Asn Gly Phe Ser Arg Ser Ser Phe Lys
            100                 105                 110

Glu Ser Leu Arg Thr Asn Phe Leu Ser Glu Val Ala Leu Lys Lys Leu
        115                 120                 125

Lys Lys Val Ser Glu Ser Gln Leu Lys Ala Ala Trp Lys Thr Tyr Gln
    130                 135                 140

Pro Lys Val Thr Val Gln His Ile Leu Thr Ser Asp Glu Asp Thr Ala
145                 150                 155                 160

Lys Gln Val Ile Ser Asp Leu Ala Ala Gly Lys Asp Phe Ala Met Leu
                165                 170                 175

Ala Lys Thr Asp Ser Ile Asp Thr Ala Thr Lys Asp Asn Gly Gly Lys
            180                 185                 190

Ile Ser Phe Glu Leu Asn Asn Lys Thr Leu Asp Ala Thr Phe Lys Asp
        195                 200                 205

Ala Ala Tyr Lys Leu Lys Asn Gly Asp Tyr Thr Gln Thr Pro Val Lys
    210                 215                 220

Val Thr Asn Gly Tyr Glu Val Ile Lys Met Ile Asn His Pro Ala Lys
225                 230                 235                 240

Gly Thr Phe Thr Ser Ser Lys Lys Ala Leu Thr Ala Ser Val Tyr Ala
                245                 250                 255

Lys Trp Ser Arg Asp Ser Ser Ile Met Gln Arg Val Ile Ser Gln Val
            260                 265                 270

Leu Lys Asn Gln His Val Thr Ile Lys Asp Lys Asp Leu Ala Asp Ala
        275                 280                 285

Leu Asp Ser Tyr Lys Lys Leu Ala Thr Thr Asn
    290                 295

<210> 5
<211> 299
<212> PRT
<213> Lactococcus lactis

<220>
<223> /note="The protease maturation protein sequence of
      Lactococcus lactis subsp. cremoris"

<400> 5

Met Lys Lys Lys Met Arg Leu Lys Val Leu Leu Ala Ser Thr Ala Thr
1               5                   10                  15

Ala Leu Leu Leu Leu Ser Gly Cys Gln Ser Asn Gln Thr Asp Gln Thr
            20                  25                  30

Val Ala Thr Tyr Ser Gly Gly Lys Val Thr Glu Ser Ser Phe Tyr Lys
        35                  40                  45

Glu Leu Lys Gln Ser Pro Thr Thr Lys Thr Met Leu Ala Asn Met Leu
    50                  55                  60

Ile Tyr Arg Ala Leu Asn His Ala Tyr Gly Lys Ser Val Ser Thr Lys
65                  70                  75                  80

Thr Val Asn Asp Ala Tyr Asp Ser Tyr Lys Gln Gln Tyr Gly Glu Asn
                85                  90                  95

Phe Asp Ala Phe Leu Ser Gln Asn Gly Phe Ser Arg Ser Ser Phe Lys
            100                 105                 110

Glu Ser Leu Arg Thr Asn Phe Leu Ser Glu Val Ala Leu Lys Lys Leu
        115                 120                 125

Lys Lys Val Ser Glu Ser Gln Leu Lys Ala Ala Trp Lys Thr Tyr Gln
    130                 135                 140

Pro Lys Val Thr Val Gln His Ile Leu Thr Ser Asp Glu Asp Thr Ala
145                 150                 155                 160

Lys Gln Val Ile Ser Asp Leu Ala Ala Gly Lys Asp Phe Ala Met Leu
                165                 170                 175

Ala Lys Thr Asp Ser Ile Asp Thr Ala Thr Lys Asp Asn Gly Gly Lys
            180                 185                 190

Ile Ser Phe Glu Leu Asn Asn Lys Thr Leu Asp Ala Thr Phe Lys Asp
        195                 200                 205

Ala Ala Tyr Lys Leu Lys Asn Gly Asp Tyr Thr Gln Thr Pro Val Lys
    210                 215                 220

Val Thr Asn Gly Tyr Glu Val Ile Lys Met Ile Asn His Pro Ala Lys
225                 230                 235                 240

Gly Thr Phe Thr Ser Ser Lys Lys Ala Leu Thr Ala Ser Val Tyr Ala
                245                 250                 255

Lys Trp Ser Arg Asp Ser Ser Ile Met Gln Arg Val Ile Ser Gln Val
            260                 265                 270

Leu Lys Asn Gln His Val Thr Ile Lys Asp Lys Asp Leu Ala Asp Ala
        275                 280                 285

Leu Asp Ser Tyr Lys Lys Leu Ala Thr Thr Asn
    290                 295

## Description

The invention relates to the field of vaccines against microbial infections and especially bacterial vaccines, in particular to pneumococcal vaccines.

*Streptococcus pneumoniae* (pneumococcus, *S. pneumoniae*) is an important pathogen, which causes significant morbidity and mortality throughout the world. *S. pneumoniae* is a major cause of invasive diseases such as meningitis, bacteremia, and pneumonia, as well as non-invasive diseases like acute otitis media and sinusitis (1). In young children, the pneumococcus is often part of the normal nasopharyngeal flora. Especially during the first two years of life, children are colonised with novel strains of pneumococci. Children colonised with *S. pneumoniae* develop more often acute otitis media than children who are not colonised (2, 3, 4).

The precise molecular mechanisms through which the pneumococcus invades and damages host tissues are not fully understood. For many years, the polysaccharide capsule has been recognised in the art as the major virulence factor and, consequently, an important vaccine candidate (for review, see 5, 6). The current pneumococcal vaccine strategies focus on the use of conjugates, in which a limited number of different capsular polysaccharides are linked to a carrier protein (7,8). Although the results of early trials look promising, problems still arise since large-scale vaccination over time generally leads to a shift in serotype distribution towards capsular types that are poorly immunogenic or not included in the vaccine. Such a shift may be enhanced by the frequent horizontal exchange of capsular genes, as described by several investigators (9, 10, 11).

Over the last few years, much attention has been focused on the role of pneumococcal proteins in pathogenesis and protection. Proteins that are involved in the pathogenesis of infections by *S. pneumoniae* are considered to be interesting components for future conjugate vaccines. Such proteins are able to switch the immune response against the polysaccharides present in the vaccine from T-cell independent to T-cell dependent, through which the antibody response towards the polysaccharides may be increased and a memory response will be provided. In addition, such proteins should provide protection against colonisation and infection with *S. pneumoniae* strains whose capsular polysaccharides are not included in the vaccine.

The protective abilities of various (virulence) proteins have been investigated previously. Immunisation of pneumolysin (12), pneumococcal surface protein A (PspA) (13, 14,15) pneumococcal surface adhesin A (PsaA) (16), and neuraminidase (17) clearly confer protection in animals.

The present invention identifies surface-associated proteins from *S. pneumoniae* with immune-protective properties. Furthermore the present invention provides the use of these proteins as vaccine components and their use in conjugate vaccination strategies.

It has now been found that a surface-associated protein of *S. pneumoniae* can be used in the preparation of a vaccine against micro-organisms and especially *S. pneumoniae*. This surface protein is present in a large number of strains of *S. pneumoniae*.

The invention accordingly relates to a vaccine or medical preparation comprising a protease maturation protein of *S. pneumoniae* and/or a fragment thereof and/or a homologous and/or functionally homologous protein and/or fragment thereof for the treatment of microbial infections and especially of *S. pneumoniae* infections. The invention also relates to the use of protease maturation protein of *S. pneumoniae* or a fragment thereof for the preparation of a vaccine for the treatment of a *S. pneumoniae* infection and/or colonisation and to the use of a protease maturation protein of *S. pneumoniae* or a fragment thereof or a recombinant or synthetic protein or fragment or functionally homologous protein or fragment thereof as a carrier for inducing prophylactic protection against other micro-organisms including viruses.

In this description and the appending claims treatment encompasses and generally is the prophylaxis of infections.

Surface-associated proteins were isolated or purified from the *S. pneumoniae* strains FT231 and EF3296, respectively, using either the SB14 extraction procedure or the Triton X114 extraction procedure as further illustrated in the working examples herein-below. The proteins and polypeptides were purified in relatively high concentrations, as shown by two-dimensional SDS-PAGE. Extracts from either strain resulted in a highly homologous protein profile as demonstrated by computer-assisted analysis. Since both extraction procedures resulted in comparable protein profiles, the SB14 extraction procedure was used for further experiments.

Hyperimmune serum antibodies were raised against the pneumococcal surface-associated proteins of *S. pneumoniae* strains FT231 and EF3296, respectively. To confirm the presence of surface-exposed proteins in the fraction, the sera were tested for the recognition of components at the surface of pneumococcal whole cells. Immuno-cytometric experiments demonstrated the recognition of components exposed at the surface of the homologous pneumococcal strains by the hyperimmune sera. Heterologous immuno-cytometric analysis demonstrated that the serum-recognition of components at the surface of the two strains display partial overlap as the level of fluorescence of the bacteria using the homologous serum was greater than the fluorescence level using the heterologous serum. In addition, components at the surface of eleven other pneumococcal strains, which display ten distinct genotypes and represent eight clinically important serotypes, were invariably recognised by the hyperimmune sera. The strains which have tested are described in more epidemiological detail by Hermans et al. (10).

Hyperimmune rabbit sera raised against the surface-associated pneumococcal proteins in the phagocytosis assay as described by Alonso Develasco et al. (5) have been analysed. The *in-vitro* opsonophagocytic activity of the serum is presumed to correlate with *in-vivo* protection against *S. pneumoniae*. The opsonophagocytic activity of the hyperimmune sera was high using the homologous pneumococcal strains. The specificity of the serum opsonophagocytic activity was determined using seven genotypically distinct pneumococcal strains, representing seven serotypes that cause most infections in young children and two strains of the genetically closely related species *S. bovis* and *Enterococcus faecalis*, respectively. The hyperimmune rabbit sera were invariably opsonically active against the pneumococcal strains. In contrast, the serum opsonophagocytic activity was very low using *S. bovis* and *E. faecalis.* This means that *S. bovis* and *E. faecalis* are not recognized by the serum. Apparently these organisms have insufficient homology to S. pneumoniae for serological recognition.

All immunodominant proteins were cut from two-dimensional acrylamide gels. Protein characterisation was performed using mass spectrometric analysis (Maldi-tof) to analyse trypsine fragments on the amino acid level. In addition, monospecific hyperimmune rabbit serum antibodies were raised against the acrylamide-embedded proteins. The monospecific hyper-immune sera were used to identify the cellular localisation of the proteins by immuno-electron microscopy and to determine the capacity of these proteins to elicit opsono-phagocytosis.

Blast and/or Blastp computer programs were used for comparison of the sequence of the protein isolated from S. pneumoniae with known sequences in various databases. In this program the Expect value (E-value) is a parameter that describes the number of hits that can be expected just by chance when searching a database. The E value is a measurement for the random background noise that exists from a match between two sequences. To decide whether or nbot a protein is functionally homologous with Pmp, a homology cutoff value is defined as an E-value of 10⁻¹⁰. A protein with an E-value of more than 10⁻¹⁰ is not considered sufficient homologous to Pmp from *S. pneumoniae.*

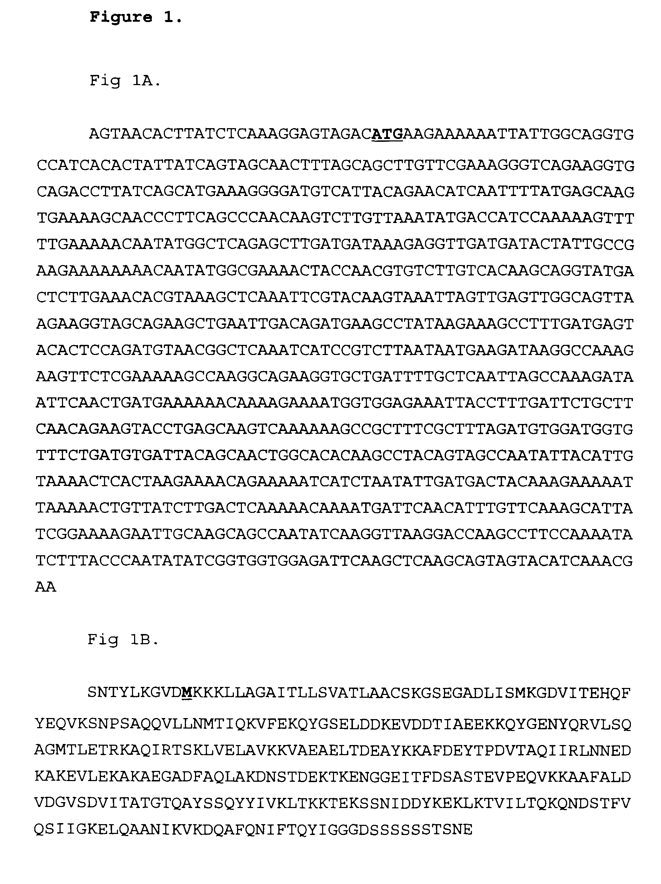

One of the proteins revealed to be homologous to a polypeptide encoded by nucleotide sequence 7632-8597 on contig 33 of *S. pneumoniae* (Figure 1). This ORF was identical to ORF 414 of *S. pneumoniae* in the WIT-system. Details about the WIT system can be found on http://wit.mcs.anl.gov/ and on the website of The Institute for Genomic Research, Rockville USA updated on April 7, 1999.

Since this pneumococcal polypeptide was related to protease maturation protein *Lactobacillus paracasei* (Swiss Prot acc. nr. Q02473) (Figure 2), and *Lactococcus lactis subspec. lactis* (Swiss Prot acc. nr. P15294) (Figure 3) and *Lactococcus lactis subsp. cremoris* (Swiss Prot acc. nr. P14308) (Figure 4) it was designated the protease maturation protein (Pmp) of *S. pneumoniae.* Also the molecular weight of the protein cut from the acrylamide gel corresponds with the molecular weight of Pmp.

This protein has various interesting characteristics with respect to its use in conjugate vaccines.

The immuno-electron microscopy using the monospecific rabbit antibodies raised against Pmp demonstrated that this protein was surface-associated.

The opsonophagocytic activity of the monospecific anti-Pmp rabbit antibodies was measured using the homologous pneumococcal strain, as well as seven genotypically distinct pneumococcal strains, representing seven serotypes that causes most infections in young children and two strains of the genetically closely related species *S. bovis* and *E.* *faecalis*, respectively. The anti-Pmp rabbit antibodies were invariably opsonically active against the pneumococcal strains. In contrast, the serum opsonophagocytic activity was very low using *S. bovis* and *E. faecalis*. These data show that Pmp has the ability to elicit immune protection, which is a major requisite with respect to its use as a vaccine component.

DNA sequence analysis of the *Pmp* genes of the homologous pneumococcal strain, as well as fifteen genotypically distinct pneumococcal strains, representing fourteen serotypes that cause most infections in young children demonstrated very limited variation. This is an important feature of Pmp with respect to its use as a vaccine component as it will guarantee immunological cross reactivity.

Phenotypic variation is an important mechanism allowing bacterial pathogens to adapt to different host environments. In *S. pneumoniae,* phenotypic variation due to alterations in cell-surface structures can be detected as spontaneous, reversible changes in colony morphology. Such alterations result in opaque and transparent colonies within single strains. The relationship of several previously identified cell-surface structures to phenotypic variation has recently been described (18). The transparent phenotype incorporates significantly more surface-exposed phosphorylcholine. In addition, the expression of three choline-binding proteins (Cbp) also varies in the phenotypic variants. The expression of autolysin LytA, is lower in opaque variants as compared to transparent variants, pneumococcal surface protein PspA is present in higher amounts in opaque variants, and CbpA is present in higher amounts in transparent variants. Such phenotypic changes also result in alterations in virulence phenotype. The opaque phenotype has decreased ability to colonise the nasopharynx as compared to the transparent phenotype (19). In addition, the survival time of mice after intraperitoneal challenge of the opaque phenotype is decreased as compared to the transparent phenotype (20)

Pmp is predominantly present in transparent colony variants of *S. pneumoniae.* Since these variants are prone to colonise the nasopharynx in animal models (21), immunisation with conjugate vaccines containing Pmp or Pmp components will enhance the removal of colonising pneumococci from the nasopharynx.

The determination of the function of Pmp in *S. pneumoniae* has been based on the homology of the protein with Pmp proteins of other bacterial species. The function of the Pmp proteins of other bacterial species is generally the activation of certain proteases. The most important keys to the use of Pmp in vaccines is the surface exposure of Pmp, whereby Pmp is available to the immunesystem and the elicitation of opsonophagocytic activity as shown in the opsonophagocytosis assay.

It being a conserved protein with surface exposure that elicits opsonophagocytic activity, enables the use of this protein and protein fragments or functional equivalents thereof in the preparation of the vaccine in such manner that the vaccine can be used against nearly all strains of *S. pneumoniae*. As Pmp is depicted as the protease maturation protein of *S. pneumoniae* and as this protein has a function as a protease activator, it is therefor easily envisaged that the protease activator proteins of other bacterial species, especially of the genus *Streptococcus,* will fulfil a major role in pathogenesis. Similar protease activators from other species, for instance from *Meisseria,* can likewise be used in vaccine preparations. These homologues and functional homologues of Pmp can thus be used in the preparation of a vaccine for other bacterial species than *S. pneumoniae.* The present invention therefor also encompasses the homologues and functional homologue equivalent proteins of Pmp and fragments and their use in vaccine preparations.

In a preferred embodiment of the invention the protein or fragments thereof used in the preparation of the vaccine, is the Pmp or a (functional) homologous fragment thereof of *S. pneumoniae* strain FT231 or strain EF 3296.

It is likewise possible to employ a fragment of Pmp for the preparation of a vaccine. A fragment is a polypeptide with an amino acid sequence which is functionally similar to the corresponding section of the protein. In principle any fragment of Pmp can be used. A preferred fragment is an oligopeptide that contains one of the characterising parts or active domains of the protein. The fragment of Pmp can be (part of) an anchoring fragment, an antigenic fragment or a fragment that is (part of) a receptor binding site or an antibody binding site or combinations thereof. The Pmp or the fragment or the functional equivalent thereof can be obtained by recombinant techniques or by chemical synthesis of Pmp oligopeptides. Synthetic oligopeptides based on or derived from Pmp can for instance be obtained by conventional pepscan technology. The use of Pmp or a (homologous) fragment or a (homologous) functional equivalent thereof as a carrier in other vaccines is also encompassed by the invention. When Pmp expresses certain strongly immunogenic properties, these properties can be used by employing Pmp as a carrier. Pmp then serves to induce an immune response to a bad immunogen such as a protein or a sugar of other bacterial or viral pathogens. This strategy is useful in conjugate vaccine strategies. In an embodiment the protease maturation protein or (homologous) fragment or (homologous) functional equivalent thereof is used as a carrier protein, preferably in a conjugate vaccine strategy.

In a preferred embodiment of the invention, the fragment is an anchoring fragment, an antigenic fragment or a functional equivalent fragment thereof or a functional equivalent for a receptor binding site or an antibody binding site.

A fragment of Pmp in general will consists of an oligopeptide of at least 5 amino acids, preferably at least about 8, but oligopeptides with 10-15 amino acids are preferred. These fragments can also be used in the from of tandem oligopeptides or dimerised oligopeptides.

The protein or (functional) fragment that is used in the preparation of the vaccine can be a partially purified protein, a purified protein or fragment of Pmp.

In order to obtain a vaccine that can be administered, the protein is brought into a form that is suitable for this purpose. To this end, the protein can be conjugated with a carrier protein. Carrier proteins that can be used in this invention are in general conventional carriers and as such are well known in the art. The vaccine can likewise also comprise adjuvants and other additional components to further ensure the proper functioning of the vaccine. These additional components are generally known by the skilled man.

In a preferred embodiment of the invention, the composition comprising the protein or the fragment is therefore combined with an adjuvant and/or a carrier. From this composition a vaccine is prepared which is used in the preventive vaccination against *S. pneumoniae.* A more preferred embodiment of the invention comprises protease maturation protein of *S. pneumoniae* or a fragment thereof for the preparation of a vaccine for the preventive treatment of a *S. pneumoniae* infection.

The invention further provides for a method for the preparation of a vaccine against *S. pneumoniae*. The method comprises the steps of preparing or isolating the protein or the fragment or homologue or functional homologue of the protein or fragment, determining the immunogenic response by raising antibodies against the protein or the fragment or homologue or functional homologue of the protein or fragment and testing the antibodies for activity. The method according to the invention also encompasses the recombinant or synthetic production of the protein or the fragment or homologue or functional homologue of the protein or fragment and the subsequent steps to the preparation of the vaccine.

In general, in this invention, when a protein or a fragment thereof is described, the protein and the fragment encompass the Pmp of *S. pneumoniae* or a fragment thereof, a homologous protein or fragment or a homologous or functional homologous protein or fragment thereof.

A preferred embodiment of the invention is a method for the preparation of a vaccine against *S. pneumoniae* comprising the steps of :
a. obtaining a protease maturation protein of *S. pneumoniae* or a fragment thereof or homologous or functionally homologous protein or fragment thereof; and
b. combining the protein or the fragment obtained under (a) with a suitable carrier or adjuvant.

The invention further provides a method for the vaccination of a mammal against an infection of *S. pneumoniae* comprising administering a suitable dose of the vaccine of the invention. The vaccine is suitable for vaccination against all strains and subspecies of *S. pneumoniae,* also for veterinary purposes.

The invention provides for the use of homologous Pmp proteins or fragments thereof of other *S. pneumoniae* species with amino acid sequences or fragments thereof such as peptides that are functionally homologous to the sequence depicted in fig 1B. Said functional homologous peptides can be used in a vaccine for the treatment, preferably the preventive treatment of a wide variety of strains and (sub)species of *S. pneumoniae.*

The sequence of the S. pneumoniae nucleotides 7632-8597 on contig 33 and the encoding polypeptide sequence harbouring Pmp are known. The nucleic acid sequence can be used to encode for Pmp or a fragment thereof. By incorporating this sequence or part thereof in a suitable vector and expressing that vector in a cell, it is possible and within the scope of the invention to obtain recombinant peptide sequences which can subsequently be used in the preparation of a vaccine.

Accordingly the invention also relates to the use of the nucleic acid sequence or fragment thereof or a (functionally) homologous sequence or fragment thereof encoding for Pmp or a fragment thereof. The invention also provides a method for the preparation of a vaccine against *S. pneumoniae.* The method comprises the principal steps of isolating the Pmp protein or the fragment thereof, determining the immunogenic response by raising antibodies against the protein or the fragment, and testing the antibodies in *S. pneumoniae* strains. The invention also provides the recombinant protein or fragment thereof, that has been obtained, for instance, through the expression of a gene sequence encoding for the protein in a suitable vector. The invention also provide for a method of obtaining an antibody and to the antibody. An embodiment of the invention is therefor a method for obtaining an antibody against protease maturation protein comprising the steps of isolating a protease maturation protein or a fragment thereof, raising antibodies against the protein or fragment thereof and isolating the antibodies. The protein or fragment that is used in the preparation of the vaccine or in obtaining the antibody can be a recombinant or synthetic protein or fragment of Pmp.

In an embodiment of the invention, the vaccine can also be derived from the expression of recombinant nucleic acids. The Pmp gene of *S. pneumoniae* can suitably be expressed in *E. coli.*

Pmp and derivatives such as fragments for instance in the form of oligopeptides and modified oligopeptides are tested in animal models to elicit the protection against the different forms of infection (otitis media, pneumonia, sepsis, meningitis) and colonisation.

The production of Pmp for vaccine purposes is in a recombinant form wherein the gene encoding for Pmp is overexpressed in gram positive and/or gram negative bacteria. This yields Pmp in bulk quantities after which further necessary steps such as purification follow.

### Description of the Figures:

Figure 1 : the *S. pneumoniae* nucleotides 7632-8597 on contig 33 (http://www.tigr.org/data/*S.pneumoniae* /) (A) and the encoding polypeptide sequence (B) harboring Pmp. The presumed methionine start codon of Pmp is depicted in bold and underscored.

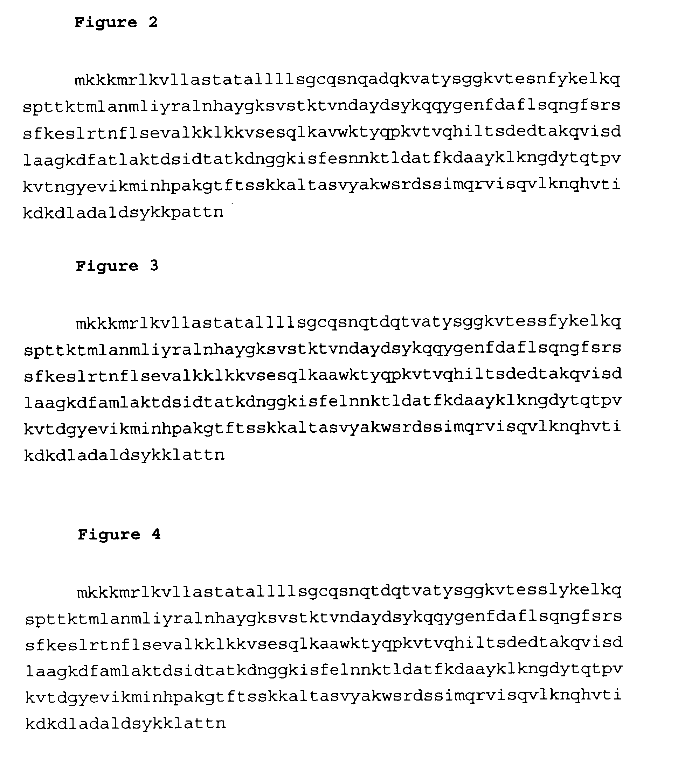

Figure 2 : The protease maturation protein of *Lactobacillus paracasei* (Swiss Prot acc. nr. Q02473).

Figure 3 : The protease maturation protein of *Lactococcus lactis subspec. lactis* (Swiss Prot acc. nr. P15294)

Figure 4 : The protease maturation protein of *Lactococcus lactis subsp. cremoris* (Swiss Prot acc. nr. P14308)

### MATERIALS AND METHODS

### Extraction of surface-associated, hydrophobic proteins of S. pneumoniae.

*S. pneumoniae* FT231 and *S. pneumoniae* EF3296 were cultured at 37 °C in Todd Hewitt broth (Difco laboratories, Detroit, USA) supplemented with 0.5% Yeast Extract (Difco laboratories). At logarithmic growth phase (OD₅₅₀=0.3) the bacteria were harvested by centrifugation, and washed three times with phosphate-buffered saline pH 7.5 (PBS). After the final washing the bacteria were resuspended in TE-buffer (10 mM Tris-Cl, 1 mM EDTA). The cells were disrupted by ultrasonic treatment (Branson sonifier 250, Branson Ultrasonics, Danburry, USA).

Extraction with sulfobetaïne 14 (SB14) was performed as described by Schouls *et al.* (22). In brief, the water-soluble cytoplasmic proteins were removed by washing the bacterial lysates five times with PBS. Cell walls, membranes and other particulate material were collected by centrifugation at 48,400*g for 20 min. Pellets were resuspended in 150 mM NaCl and centrifuged for 20 min at 48,400*g. The pellets were then incubated for 2 hours at room temperature with 0.25% N-tetradecyl-N,N-dimetylammonio-1-propanesulfonate (SB14, Serva, Heidelberg, Germany) in the presence of 150 mM NaCl, 10 mM MgCl₂ and 10 mM Tris-HCl pH 8.0 during constant stirring. The hydrophobic, membrane-associated proteins were recovered as described by Wessel and Flügge (23).

Extraction with Triton X114 (Sigma, St. Louis, USA) was also performed as described by Schouls *et al.* (24). Briefly, bacterial lysates were centrifuged at 20,000*g for 20 min. Pellets were dissolved with 1% Triton X114 in PBS for 1 hour at 0 °C. After extraction, the suspensions were centrifuged at 25,000* g at 4 °C for 1 hour, the supernatants were incubated at 37 °C for 30 min, and centrifuged at 25,000*g at 25 °C for 1 hour to separate the detergent phase and aqueous phase. The proteins in the detergent phase were extracted according to the procedure of Wessel and Flügge (23). Protein concentrations were measured by the method of Bradford (25).

### Protein electrophoresis and staining.

One-dimensional sodium dodecyl sulfate polyacrylamide gel electrophoresis (SDS-PAGE) was carried out in the Biorad minigel system with 13% polyacrylamide gels. The samples were dissolved in sample buffer (10 mM Tris-HCl, 1 mM EDTA, 1% SDS, 10 mM DTT, 1% glycerol, 0.01% bromophenol blue indicator (Merck, Darmstadt, Germany)), boiled for 5 min and subjected to electrophoresis (26).

Two-dimensional SDS-PAGE was performed according to the instructions of the manufacturer (Pharmacia Biotech, Uppsala, Sweden) including modifications of Rabilloud *et al.* (27). After isoelectric focusing, proteins were separated using gradient (12-20%) polyacrylamide gel electrophoresis.

Silver staining of polyacrylamide gels was performed as described by Blum *et al.* (28). In addition, standard procedures were used to stain the polyacrylamide gels using Coomassie brilliant blue (CBB) (26).

The software program PD Quest (PDI, New York, USA) was used for the computerized analysis of two-dimensional SDS-PAGE gels.

### Hyperimmune rabbit antiserum.

Hyperimmune antiserum was raised against the hydrophobic, surface-associated proteins by injecting New-Zealand White rabbits subcutaneously into 4-5 places. The SB14 and Triton X114-extracted hydrophobic surface-associated proteins (500 µg) of *S. pneumoniae* FT231 and EF3296, respectively, were dissolved in 0.5 ml 0.9% NaCl, and subsequently mixed with 0.5 ml Freund's incomplete adjuvant (Pierce, Rockford, USA). In addition, hyperimmune rabbit serum was raised against SB14-purified hydrophobic surface-associated proteins of *S. pneumoniae* FT231 that were subjected to 1D-SDS-PAGE. The total protein pool was cut from the polyacrylamide gel, washed three times with 0.1 M NaAc, 96% EtOH, ground into a fine suspension in 0.5 ml PBS, and subsequently mixed with 0.5 ml Freund's incomplete adjuvant. Negative control serum was gained by injection of washed and ground polyacrylamide in 0.5 ml PBS mixed with 0.5 ml Freund's incomplete adjuvant. The primary injection was followed by four subcutaneous booster injections at four-week intervals.

Antibodies to type 2 capsule were purchased from Statens Seruminstitut, Copenhagen, Denmark. Recombinant pneumolysin was used to raise hyperimmune sera in rabbits as described previously (29). These sera were used as positive controls in passive immunization experiments.

### Indirect immuno-cytometric assay.

Pneumococci were grown to logarithmic phase in Todd-Hewitt broth supplemented with 0.5% Yeast Extract at 37 °C using 5% CO₂, then washed three times in ice-cold PBS and stored overnight at 4 °C. The bacteria were incubated in 5% rabbit serum (10⁷ bacteria in 20 µl final volume) for 15 min at 4 °C while shaking. The bacteria were washed twice using ice-cold PBS and incubated for 15 min at 4 °C with 20 µl (1:5 dilution) of fluorescein-conjugated goat anti-rabbit IgG (Jackson ImmunoResearch Laboratories, West Grove, USA) while shaking. The bacteria were washed twice with ice-cold PBS and resuspended in 100 µl of ice-cold paraformaldehyde (0.5 %) in PBS. The samples were analyzed in a FACScan flow cytometer (Becton Dickinson, Mountain View, USA).

### Phagocytosis assay.

Analysis of the opsonophagocytic activity of the sera was performed as described by Alonso Develasco *et al.* (30). In brief, *S. pneumoniae* was grown to logarithmic phase in Todd-Hewitt broth supplemented with 0.5% Yeast Extract at 37 °C using 5% CO₂. After washing with PBS, the bacteria were labeled with fluorescein-isothiocyanate (FITC, isomer I, Sigma Chemical Co., St. Louis, USA) (0.5 mg/ml in PBS) for 1 hour at 4 °C, washed twice and resuspended in Hank's balanced salt solution (HBSS) containing 1% w/v bovine BSA. The bacteria (10⁸ bacteria per 100 µl BSA-HBSS) were stored at -20 °C. Samples of 2.5 * 10⁶ bacteria were transferred into round-bottom microtiter plates (Greiner Labortechnik, Alphen a/d Rijn, The Netherlands). Rabbit sera diluted in BSA-HBSS and heat-inactivated for 30 min at 56 °C were added to the bacteria (final volume 50 µl). The opsonization was performed at 37 °C for 30 min while shaking. Plates were then placed on ice and 2.5 * 10⁵ human polymorphonuclear cells isolated from peripheral blood of healthy volunteers were added to each well (final volume 100 µl). Human PMNs were isolated by mixing 30 ml of heparinized blood with 30 ml of phosphate-buffered saline (pH 7.4), layered on Ficoll-Paque, and centrifuged for 20 min at 400*g. The lowest layer containing PMNs and erythrocytes was washed once in RPMI (Gibco BRL, Life Technologies LTD, Paisley, UK) containing 0,05% human serum albumin. The erythrocytes were lysed using ice-cold lysis buffer (155 mM NH₄Cl, 10 mM KHCO₃, 1 mM EDTA, pH 7.4). Phagocytosis was performed for 30 min at 37 °C while shaking. After washing twice with ice-cold HBSS, samples were resuspended in 200 µl of HBSS. The PMNs were fixed by adding 100 µl PBS-2% paraformaldehyde, and the samples were analyzed in a FACScan flow cytometer (Becton Dickinson). Fluorescent PMNs observed after opsonization with antiserum indicates both uptake and binding (referred to as phagocytosis) of FITC-labeled bacteria. The opsonophagocytic activity is defined as the reciprocal of the serum concentration at which 25 % of the human PMNs were fluorescent.

### Immuno electron spectroscopy.

Immuno electron spectroscopy was performed according to the standard operational procedures of the national institute for biological standards and control, Potters bar, United Kinmgdom.

### References

1 Caputo, G. M., M. Singer, S. White, and M. R. Weitekamp. 1993. Infections due to antibiotic-resistant gram-positive cocci. J. Gen. Intern. Med. **8**:626-634.
2 Faden, H., L. Duffy, R. Wasielewski, J. Wolf, D. Krystofik, and Y. Tung. 1997. Relationship between nasopharyngeal colonisation and the development of otitis media in children; Tonawanda/Williamsville Pediatrics. J Infect Dis. 175:1440-1445
3 Homoe, P., J. Prag, S. Farholt, J. Henrichsen, A. Hornsleth, M. Kilian, and J. S. Jensen. 1996. High rate of nasopharyngeal carriage of potential pathogens among children in Greenland: results of a clinical survey of middle-ear disease. Clin Infect Dis. 23:1081-1090.
4 Zenni, M. K., S. H. Cheatham, J. M. Thompson, G. W. Reed, A. B. Batson, P. S. Palmer, K. L. Holland, and K. M. Edwards. 1995. *S. pneumoniae* colonisation in the young child: association with otitis media and resistance to penicillin. J Pediatr. **127**:533-537
5 Alonso Develasco E, Verheul AF, Verhoef J, Snippe H. S. pneumoniae : virulence factors, pathogenesis, and vaccines. Microbiol Rev 1995;59:591-603.
6 Mitchell TJ, Alexander JE, Morgan PJ, Andrew PW. Molecular analysis of virulence factors of *S. pneumoniae* . Soc. Appl. Bacteriol. Symp. Ser. 1997;26:62S-71S.
7 Butler JC. Epidemiology of pneumococcal serotypes and conjugate vaccine formulations. Microb. Drug Resist. 1997;3:125-9
8 Dagan R, Melamed R, Muallem M, Piglansky L, Yagupsky P. Nasopharyngeal colonisation in southern Israel with antibiotic-resistant pneumococci during the first 2 years of life: relation to serotypes likely to be included in pneumococcal conjugate vaccines. J. Infect. Dis. 1996;174:1352-5.
9 Barnes DM, Whittier S, Gilligan PH, Soares S, Tomasz A, Henderson FW. Transmission of multidrug-resistant serotype 23F *S. pneumoniae* in group day care: evidence suggesting capsular transformation of the resistant strain *in vivo*. J. Infect. Dis. 1995;171:890-6.
10 Hermans PW, Sluijter M, Dejsirilert S, et al. Molecular epidemiology of drug-resistant pneumococci: toward an international approach. Microb. Drug Resist. 1997;3:243-51.
11 Hermans PW, Sluijter M, Elzenaar K, et al. Penicillin-resistant *S. pneumoniae* in the Netherlands: results of a 1-year molecular epidemiologic survey. J. Infect. Dis. 1997;175:1413-22.
12 Paton JC, Lock RA, Hansman DJ. Effect of immunisation with pneumolysin on survival time of mice challenged with *S. pneumoniae* . Infect. Immun. 1983;40:548-52.
13 McDaniel LS, Sheffield JS, Delucchi P, Briles DE. PspA, a surface protein of *S. pneumoniae* , is capable of eliciting protection against pneumococci of more than one capsular type. Infect. Immun. 1991;59:222-8.
14 Talkington DF, Crimmins DL, Voellinger DC, Yother J, Briles DE. A 43-kilodalton pneumococcal surface protein, PspA: isolation, protective abilities, and structural analysis of the amino-terminal sequence. Infect. Immun. 1991;59:1285-9.
15 Wu MHN, Y. Guo, Michael W. Russel, and David E. Briles. Intranasal immunisation of mice with pspA (pneumococcal surface protein A) can prevent intranasal carriage, pulmonary infection, and sepsis with *S. pneumoniae .* J. Infect. Dis. 1997;175:839-846.
16 Talkington DF, Brown BG, Tharpe JA, Koenig A, Russell H. Protection of mice against fatal pneumococcal challenge by immunisation with pneumococcal surface adhesin A (PsaA). Microb. Pathog. 1996;21:17-22.
17 Lock RA, Paton JC, Hansman D. Comparative efficacy of pneumococcal neuraminidase and pneumolysin as immunogens protective against *S. pneumoniae* . Microb. Pathog. 1988;5:461-7.14.
18 Weisser, J.N. Phase variation in colony opacity by S. pneumoniae . Microbial Drug Resistance 4(1998):129-135. 19 Weisser, J.N., Austrian, R., Sreenivasan, P.K., Masure, H.R. Phase variation in pneumococcal opacity: relationship between colonial morphology and nasopharyngeal colonisation. Infection and Immunity 62(1994):2582-2589.
20 Kim, J.O., Weiser, J.N. Association of intrastrain phase variation in quantity of capsular polysaccharide and teichoic acid with the virulence of *S. pneumoniae .* Journal of Infectious Diseases 177(1998):368-377.
21 Weiser JN, Markiewicz, Z, Tuomanen, EI, Wani, JH. Relationship between phase variation in colony morphology, intrastrain variation in cell wall physiology, and nasopharyngeal colonisation by *S. pneumoniae .* Infect. Immun. 1996; 64:2240-2245.
22 Schouls LM, Ijsselmuiden OE, Weel J, van Embden JD. Overproduction and purification of *Treponema pallidum* recombinant-DNA-derived proteins TmpA and TmpB and their potential use in serodiagnosis of syphilis. Infect. Immun. 1989;57:2612-23.
23 Wessel D, Flugge UI. A method for the quantitative recovery of protein in dilute solution in the presence of detergents and lipids. Anal. Biochem. 1984;138:141-3.
24 Schouls LM, van der Heide HG, van Embden JD. Characterization of the 35-kilodalton *Treponema pallidum subsp. pallidum* recombinant lipoprotein TmpC and antibody response to lipidated and nonlipidated *T. pallidum* antigens. Infect. Immun. 1991;59:3536-46.
25 Bradford MM. A rapid and sensitive method for the quantitation of microgram quantities of protein utilizing the principle of protein-dye binding. Anal. Biochem. 1976;72:248-54.
26 Sambrook J, Fritsch EF, Maniatis T. Molecular Cloning. A laboaratory manual. 2nd edn. Cold Spring Harbor Laboratory Press, 1989.
27 Rabilloud T, Valette C, Lawrence JJ. Sample application by in-gel rehydration improves the resolution of two-dimensional electrophoresis with immobilized pH gradients in the first dimension. Electrophoresis 1994;15:1552-8.
28 Blum H, Beier H, Gross HJ. Improved silver staining of plant proteins, RNA and DNA in polyacrylamide gels. Electrophoresis 1987;8:93-99.
29 Mitchell T, Walker J, Saunders F, Andrew P, GJ B. Expression of the pneumolysin gene in *Escherichia coli:* rapid purification and biological properties. Biochim. Biophys. Acta 1987;1007: 67-72.
30 Alonso Develasco E, Verheul AFM, van Steijn AMP, Dekker HAT, Feldman RG, Fernandez IM, Kamerling JP, Vliegenthart JFG, Verhoef J and Snippe H. 1994. Epitope specificity of rabbit elicited by pneumococcal type 23F synthetic oligosaccharide- and native polysaccharide-protein conjugate vaccines: comparison with human anti-polysaccharide 23F IgG. Infect. Immun. 62: 799-808

## Claims

1. A vaccine or medical preparation comprising a protease maturation protein of *S. pneumoniae* and/or a fragment thereof and/or a homologous and/or a functionally homologous protein or protein fragment thereof for the treatment of microbial infections.

2. The vaccine or medical preparation according to claim 1 for the treatment of *S. pneumoniae.*

3. The vaccine or medical preparation according to claim 1 or 2, further comprising a suitable adjuvant or carrier.

4. The vaccine or medical preparation according to anyone of the claims 1-3, wherein said protein comprises an amino acid sequence as shown in fig 1B.

5. The vaccine or medical according to anyone of the claims 1-4, wherein said protein is the protein maturation protein from *S. pneumoniae* Ft231 or EF3296.

6. The vaccine or medical preparation according to anyone of the claims 1-5, wherein said fragment comprises an anchoring fragment, an antigenic fragment or a functional equivalent thereof or a functional equivalent of a receptor binding site or an antibody binding site.

7. The vaccine or medical preparation according to anyone of the claims 1-6, wherein said protein or said fragment comprises a purified, partly purified, recombinant or synthetic protein or fragment thereof.

8. The vaccine or medical preparation according to anyone of the claims 1-7, wherein said fragment comprises at least 8 amino acids.

9. Method for the preparation of a vaccine against *S. pneumoniae* comprising the steps of:
a. isolating a protease maturation protein of *S. pneumoniae* or a fragment thereof or a recombinant or synthetic protein or fragment thereof or homologous or functionally homologous protein or fragment thereof; and
b. combining the protein or the fragment thereof obtained under (a) with a suitable carrier or adjuvant.

10. Method for obtaining an antibody against the protease maturation protein of *S. pneumoniae*, comprising the steps of isolating said protease maturation protein or a fragment thereof, and raising antibodies against said protein or fragment thereof.

11. Antibody obtainable by the method according to claim 10.

12. Use of a protease maturation protein of *S. pneumoniae* or a fragment thereof for the preparation of a vaccine for the treatment or prophylaxis of a *S. pneumoniae* infection.

13. Use of a protease maturation protein of *S. pneumoniae* or a fragment thereof or a recombinant or synthetic protein or fragment thereof as a carrier.

14. Method of treatment of a *S. pneumoniae* infection comprising administering a vaccine according to claims 1-8.

15. Method for the vaccination of a mammal against an infection of *S. pneumoniae* comprising administering a suitable dose of a vaccine according to anyone of the claims 1-8.

16. Use of a nucleic acid sequence encoding for a protease maturation protein or a fragment thereof for obtaining a recombinant protease maturation protein or fragment thereof.

17. Cell containing a recombinant nucleic acid sequence or a vector encoding for protease maturation protein or a fragment thereof.

18. Recombinant protease maturation protein or fragment thereof obtainable through the expression of a gene sequence encoding for said protein in a suitable vector.
